# EUROPEAN PATENT APPLICATION

(11) **EP 1 118 680 A1**
(43) Date of publication of application: **25.07.2001**
(21) Application number: 01300272.0
(22) Date of filing: 12.01.2001
(51) Int. Cl.: C12Q 1/68

(54) **Method for the diagnosis of polymorphisms in the human PDH E1alpha gene**

(30) Priority: 18.01.2000 GB 0001005
(71) Applicant: AstraZeneca AB, 151 85 Södertälje (SE)
(72) Inventor: Smith, John Craig, AstraZeneca, Macclesfield, Cheshire SK10 34TG (GB); Anand, Rakesh, AstraZeneca, Macclesfield, Cheshire SK10 4TG (GB); Morten, John Edward Norris, AstraZeneca, Macclesfield, Cheshire SK10 4TG (GB)
(74) Representative: Giles, Allen Frank

(57) **Abstract**

This invention relates to polymorphisms and novel sequence in the human pyruvate dehydrogenase E1α (PDH E1α) gene. The invention also relates to methods and materials for analysing allelic variation in the PDH E1α gene, and to the use of PDH E1α polymorphism in the diagnosis and treatment of diseases in which modulation of pyruvate dehydrogenase activity could be of therapeutic benefit, such as diabetes, asthma, obesity, sepsis and peripheral vascular disease. In particular, the invention is based on the discovery of a nucleotide polymorphism in the 3' untranslated region (3'UTR) of the human PDH E1α gene. In addition, we disclose the sequence of intron 7 of the human PDH E1α gene and identify two polymorphisms within intron 7.

SEQUENCE LISTING

<110> AstraZeneca AB
      Morten, John R
      Smith, John C
      Anand, Rakesh

<120> Methods

<130> PDH Elalpha

<140>
<141>

<160> 2

<170> PatentIn Ver. 2.1

<210> 1
<211> 175
<212> DNA
<213> Homo sapiens

<400> 1
gtaaggacac ctgtggtggg gccggggcca aggccaaggg tatgtccttg tgcagaccct 60
tgacgatctt agaaacattg gagagtttca ttctcataca ggagcaggtc atgtgaaagt 120
aaaatggttt ggggcagttg gattcatgct tcgcccctcc cctgtttatt accag 175

<210> 2
<211> 1472
<212> DNA
<213> Homo sapiens

<400> 2
actgaggcgt ggcgtctgct ggggcacctg aaggagactt gggggcaccc gcgtcgtgcc 60
tcctgggttg tgaggagtcg ccgctgccgc cactgcctgt gcttcatgag gaagatgctc 120
gccgccgtct cccgcgtgct gtctggcgct tctcagaagc cggcaagcag agtgctggta 180
gcatcccgta attttgcaaa tgatgctaca tttgaaatta agaaatgtga ccttcaccgg 240
ctggaagaag gccctcctgt cacaacagtg ctcaccaggg aggatgggct caaatactac 300
aggatgatgc agactgtacg ccgaatggag ttgaaagcag atcagctgta taaacagaaa 360
attattcgtg gtttctgtca cttgtgtgat ggtcaggaag cttgctgtgt gggcctggag 420

gccggcatca accccacaga ccatctcatc acagcctacc gggctcacgg ctttactttc 480
acccggggcc tttccgtccg agaaattctc gcagagctta caggacgaaa aggaggttgt 540
gctaaaggga aaggaggatc gatgcacatg tatgccaaga acttctacgg gggcaatggc 600
atcgtgggag cgcaggtgcc cctgggcgct gggattgctc tagcctgtaa gtataatgga 660
aaagatgagg tctgcctgac tttatatggc gatggtgctg ctaaccaggg ccagatattc 720
gaagcttaca acatggcagc tttgtggaaa ttaccttgta ttttcatctg tgagaataat 780
cgctatggaa tgggaacgtc tgttgagaga gcggcagcca gcactgatta ctacaagaga 840
ggcgatttca ttcctgggct gagagtggat ggaatggata tcctgtgcgt ccgagaggca 900
acaaggtttg ctgctgccta ttgtagatct gggaaggggc ccatcctgat ggagctgcag 960
acttaccgtt accacggaca cagtatgagt gaccctggag tcagttaccg tacacgagaa 1020
gaaattcagg aagtaagaag taagagtgac cctattatgc ttctcaagga caggatggtg 1080
aacagcaatc ttgccagtgt ggaagaacta aaggaaattg atgtggaagt gaggaaggag 1140
attgaggatg ctgcccagtt tgccacggcc gatcctgagc cacctttgga agagctgggc 1200
taccacatct actccagcga cccacctttt gaagttcgtg gtgccaatca gtggatcaag 1260
tttaagtcag tcagttaagg ggaggagaag gagaggttat accttcaggg ggctaccaga 1320
cagtgttctc aacttggtta aggaggaaga aaacccagtc aatgaaattc aatgaaattc 1380
ttggaaactt ccattaagtg tgtagattga gcaggtagta attgcatgca gtttgtacat 1440
tagtgcatta aaagatgaat tattgagtgc tt 1472

## Description

This invention relates to polymorphisms and novel sequence in the human pyruvate dehydrogenase E1α (PDH E1α) gene. The invention also relates to methods and materials for analysing allelic variation in the PDH E1α gene, and to the use of PDH E1α polymorphism in the diagnosis and treatment of diseases in which modulation of pyruvate dehydrogenase activity could be of therapeutic benefit, such as diabetes, asthma, obesity, sepsis and peripheral vascular disease.

The production of energy for biosynthesis of complex molecules and for muscle contraction is mediated by the hydrolysis of high energy phosphate bonds within adenosine triphosphate (ATP). In oxidative metabolism ATP is generated from acetyl coenzyme A (acetyl CoA), which itself is produced by the beta-oxidation of fatty acids, or as a result of the metabolism of glucose via the glycolytic pathway. The key regulatory enzyme which controls the rate of acetyl CoA formation from glucose is pyruvate dehydrogenase (PDH), which catalyses the oxidation of pyruvate to acetyl CoA and carbon dioxide with concomitant reduction of NAD to NADH.

PDH is a multienzyme complex located in the mitochondrial matrix, containing multiple copies of three enzyme components required to complete the conversion of pyruvate to acetyl CoA (Patel and Roche 1990; FASEB J., 4: 3224-3233). E1 (pyruvate decarboxylase, E.C. 1.2.4.1) catalyses the non-reversible removal of carbon dioxide from pyruvate; E2 (dihydrolipoamide acetyltransferase, E.C. 2.3.1.12) catalyses the formation of acetyl CoA; and E3 (dihydrolipoamide dehydrogenase, E.C. 1.8.1.4) reduces NAD to NADH. The E1 enzyme is a heterotetramer composed of two α and two β subunits. Decarboxylation of pyruvate, catalysed by El is the rate limiting step in the overall activity of the PDH complex. This step is also the target for a cycle of phosphorylation and dephosphorylation which forms one of the main mechanisms for regulating PDH activity. Two additional enzyme activities are also associated with the PDH complex: a specific kinase (PDK) which is capable of phosphorylating E1α at three serine residues, and a loosely-associated specific phosphatase which reverses the phosphorylation. Phosphorylation of only one of the three serine residues on E1α renders E1 inactive. Removal of the phosphate groups by the specific phosphatase restores activity. Thus, the proportion of PDH in its active (dephosphorylated) state is determined by a balance between the activity of the kinase and phosphatase. The activity of the kinase may be regulated in vivo by the relative concentrations of metabolic substrates such as NAD/NADH, CoA/acetylCoA and ADP/ATP as well as by the availability of pyruvate itself, therefore providing highly regulated, responsive control of PDH activity.

Genetic abnormalities in the PDH complex are the most common cause of primary lactic acidosis in humans. The majority of cases have been linked with a defect in the E1α subunit. Pathologies associated with defects in the PDH complex conform to a broad clinical spectrum ranging from fatal lactic acidosis in the newborn, to a range of chronic neurodegenerative conditions with gross structural abnormalities in the central nervous system. E1α deficiency is an X-linked disorder which manifests different patterns of clinical presentation between males and females. In addition, heterozygous females show a wide variation in clinical severity of the disease, due largely to variations in the pattern of X-inactivation and differential effects of specific gene mutations on the expression, stability and activity of the mutant protein. A number of mutations in the PDH gene which lead to pyruvate dehydrogenase deficiency have been documented (for a review see NIH OMIM database, reference 312170).

In disease states such as both non-insulin dependent (NIDDM) and insulin-dependent diabetes (IDDM), oxidation of lipids is increased with a concomitant reduction in utilisation of glucose, contributing to the hyperglycaemia. The activity of PDH is reduced in both insulin-dependent and non insulin-dependent diabetes. A further consequence of reduced PDH activity is an increase in pyruvate concentration resulting in increased availability of lactate as a substrate for hepatic gluconeogenesis. Diabetes is further exacerbated by impaired insulin secretion, which has been shown to be associated with reduced PDH activity in pancreatic β-cells. It is believed that increasing the activity of PDH may increase the rate of glucose oxidation and hence overall glucose utilisation, in addition to reducing hepatic glucose output.

Oxidation of glucose is capable of yielding more molecules of ATP per mole of oxygen than is oxidation of fatty acids, therefore in conditions where energy demand may exceed energy supply, such as myocardial ischaemia and reperfusion, intermittent claudication, cerebral ischaemia and reperfusion, shifting the balance of substrate utilisation in favour of glucose metabolism may be expected to improve the ability to maintain ATP levels and hence function. Activation of PDH is predicted to have this effect.

An agent which is capable of activating PDH is expected to be of benefit in treating conditions where an excess of circulating lactic acid is manifest such as in certain cases of sepsis.

The agent dichloroacetic acid which increases the activity of PDH after acute administration in animals (Vary et al., 1988; Circ. Shock, 24: 3-18) has been shown to have the predicted effects in reducing glycaemia (Stacpoole et al, 1978 N. Engl. J. Med. 298, 526-530) and as a therapy for myocardial ischaemia (Bersin and Stacpoole 1997; American Heart Journal, 134: 841-855) and lactic acidaemia (Stacpoole et al, 1983 N. Engl. J. Med 309, 390-396).

cDNA sequences encoding PDH E1α have been submitted to public databases under the following accession numbers: L13318, J02734, J03503, X52709, X52710, J03575, M24848, L48690, D90084, M58568. All positions in the coding region and 3' untranslated region (3'UTR) of the human PDH E1α gene herein refer to the positions in SEQ ID NO: 2 (which was EMBL accession number L13318 at the time of filing) unless stated otherwise or apparent from the context.

In the present invention we disclose the sequence of intron 7 of the human PDH Elα gene. All positions in intron 7 of the human PDH E1α gene herein refer to the positions disclosed in SEQ ID NO.1 unless stated otherwise or apparent from the context.

The structural organisation of the PDH Elα gene has been published by Maragos C. *et al.*, J Biol Chem 264, 12294-12298, 1989. The gene is located at Xp22.2-22.1 (Borglum A.D. *et al.*, Hum. Genet. 99, 80-82, 1997). Dahl et al (1991) Hum Genet, **87**:49-53 have published a silent polymorphism at position 900 of the coding region and microsatellite (CA)ₙ repeats.

DNA polymorphisms may lead to variations in amino acid sequence and consequently to altered protein structure and functional activity. Polymorphisms may also affect mRNA synthesis, maturation, transportation and stability. Polymorphisms which do not result in amino acid changes (silent polymorphisms) or which do not alter any known consensus sequences may nevertheless have a biological effect, for example by altering mRNA folding or stability.

Knowledge of polymorphisms may be used to help identify patients most suited to therapy with particular pharmaceutical agents (this is often termed "pharmacogenetics"). Pharmacogenetics can also be used in pharmaceutical research to assist the drug selection process. Polymorphisms may be used in mapping the human genome and to elucidate the genetic component of diseases. The reader is directed to the following references for background details on pharmacogenetics and other uses of polymorphism detection: Linder *et al.* (1997), Clinical Chemistry, 43, 254; Marshall (1997), Nature Biotechnology, 15, 1249; International Patent Application WO 97/40462, Spectra Biomedical; and Schafer *et al.* (1998), Nature Biotechnology, 16, 33.

Clinical trials have shown that patient response to treatment with pharmaceuticals is often heterogeneous. Thus there is a need for improved approaches to pharmaceutical agent design and therapy.

The present invention is based on the discovery of a nucleotide polymorphism in the 3' untranslated region (3'UTR) of the human PDH E1α gene. In addition, we disclose the sequence of intron 7 of the human PDH E1α gene and identify two polymorphisms within intron 7.

According to one aspect of the present invention there is provided a method for the diagnosis of a polymorphism in a PDH E1α gene in a human, which method comprises determining the sequence of the nucleic acid of the human at position 1388 in the PDH Elα gene as defined by the position in SEQ ID NO: 2, and/or at one or more of positions 26 and 161 of intron 7 of the PDH Elα gene as defined in SEQ ID NO.1; and determining the status of the human by reference to polymorphism in the PDH Elα gene.

The term human includes both a human having or suspected of having a PDH-mediated disease and an asymptomatic human who may be tested for predisposition or susceptibility to such disease. At each position the human may be homozygous for an allele or the human may be a heterozygote.

The term 'PDH-mediated disease' means any disease in which changing the level of PDH or changing the activity of PDH would be of therapeutic benefit.

The term 'PDH drug' means any drug which changes the level of PDH or changes the activity of PDH. A drug which increases the activity of PDH is preferred.

The term polymorphism includes single nucleotide substitution, nucleotide insertion and nucleotide deletion, which in the case of insertion and deletion includes insertion or deletion of one or more nucleotides at a position of a gene and variable numbers of a repeated DNA sequence.

In another embodiment of the invention preferably the method for diagnosis described herein is one in which the single nucleotide polymorphism at position 1388 in the PDH Elα gene as defined by the position in SEQ ID NO: 2 is presence of C and/or T.

In another embodiment of the invention preferably the method for diagnosis described herein is one in which the polymorphism at position 26 of intron 7 of the PDH Elα gene as defined by the positions in SEQ ID NO.1 is the presence or absence of a hexanucleotide repeat (GGCCAA)n. Preferably n = 0 - 100. More preferably n = 0 - 20. More preferably n = 1 - 10. More preferably n = 2, 3 or 4. In the sequence shown in SEQ ID NO.1, n = 2. The most common allele in the population studied was n = 3 (see Example 1).

In another embodiment of the invention preferably the method for diagnosis described herein is one in which the single nucleotide polymorphism at position 161 of intron 7 of the PDH Elα gene as defined by the positions in SEQ ID NO.1 is presence of C and/or A.

A at position 161 of intron 7 was found to be strongly associated with a hexanucleotide repeat (GGCCAA)n where n = 2 at position 26 of intron 7 as defined by the positions in SEQ ID NO.1 (see Example 1).

It will be appreciated by the person skilled in the art that the numbering of the nucleotide positions in intron 7 of the PDH Elα gene will vary according to the number of hexanucleotide repeats at position 26 as defined by the sequence in SEQ ID NO.1. For example, in SEQ ID NO.1 the number of hexanucleotide repeats at position 26 is two, thereby placing the corresponding position of the intron 7 single nucleotide polymorphism at position 161. When the number of hexanucleotide repeats is three, the corresponding position of the intron 7 single nucleotide polymorphism will be 167. When the number of hexanucleotide repeats is four, the corresponding position of the intron 7 single nucleotide polymorphism will be 173 and so forth.

Preferably the polymorphisms of the invention are further defined as:

| **Position** | **Reference** | **Region** | **Polymorphism** |
|---|---|---|---|
| 26 | SEQ ID NO: 1 | intron 7 | (GGCCAA)n |
| 161 | SEQ ID NO:1 | intron 7 | C/A |
| 1388 | SEQ ID NO:2 | 3' UTR | C/T |

A preferred method of the invention comprises diagnosis of the following haplotype:

| **Position** | **Reference** | **Region** | **Polymorphism** |
|---|---|---|---|
| 26 | SEQ ID NO:1 | intron 7 | (GGCCAA)₂ |
| 161 | SEQ ID NO:1 | intron 7 | A |

The method for diagnosis of a single nucleotide polymorphism is preferably one in which the sequence is determined by amplification refractory mutation system. The method for diagnosis of a hexanucleotide repeat is preferably one in which the sequence is determined by polyacrylamide gel electrophoresis or capillary electrophoresis.

In another aspect of the invention we provide a method for the diagnosis of PDH-mediated disease, which method comprises:
i) obtaining sample nucleic acid from an individual,
ii) detecting the presence or absence of a variant nucleotide at position 1388 in the PDH Elα gene as defined by the position in SEQ ID NO: 2 and/or at one or more of positions 26 and 161 of intron 7 of the PDH Elα gene as defined by the positions in SEQ ID NO.1; and
iii) determining the status of the individual by reference to polymorphism in the PDH E1α gene.

Allelic variation at position 1388 in the PDH Elα gene as defined by the positions in SEQ ID NO: 2 consists of a single base substitution from C (the published base), preferably to T. Allelic variation at position 26 of intron 7 of the PDH E1α gene as defined by the positions in SEQ ID NO.1 consists of insertion of a hexanucleotide repeat (GGCCAA)n. Preferably n = 0 - 100. More preferably n = 0 - 20. More preferably n = 1 - 10. More preferably n = 2, 3 or 4. Allelic variation at position 161 of intron 7 of the PDH Elα gene as defined by the positions in SEQ ID NO.1 consists of a single base substitution from C (the base shown in SEQ ID NO.1) to A. The status of the individual may be determined by reference to allelic variation at any one, two, three or all four positions optionally in combination with any other polymorphism in the gene that is (or becomes) known.

The test sample of nucleic acid is conveniently present in a sample of blood, sputum, skin, bronchoalveolar lavage fluid, or other body fluid or tissue obtained from an individual. It will be appreciated that the test sample may equally comprise a nucleic acid sequence corresponding to the sequence in the test sample, that is to say that all or a part of the region in the sample nucleic acid may firstly be amplified using any convenient technique e.g. PCR, before analysis of allelic variation.

It will be apparent to the person skilled in the art that there are a large number of analytical procedures which may be used to detect the presence or absence of variant nucleotides at one or more polymorphic positions of the invention. In general, the detection of allelic variation requires a mutation discrimination technique, optionally an amplification reaction and optionally a signal generation system. Table 1 lists a number of mutation detection techniques, some based on PCR. These may be used in combination with a number of signal generation systems, a selection of which is listed in Table 2. Further amplification techniques are listed in Table 3. Many current methods for the detection of allelic variation are reviewed by Nollau *et al.,* Clin. Chem. 43, 1114-1120, 1997; and in standard textbooks, for example "Laboratory Protocols for Mutation Detection", Ed. by U. Landegren, Oxford University Press, 1996 and "PCR", 2^{nd} Edition by Newton & Graham, BIOS Scientific Publishers Limited, 1997.

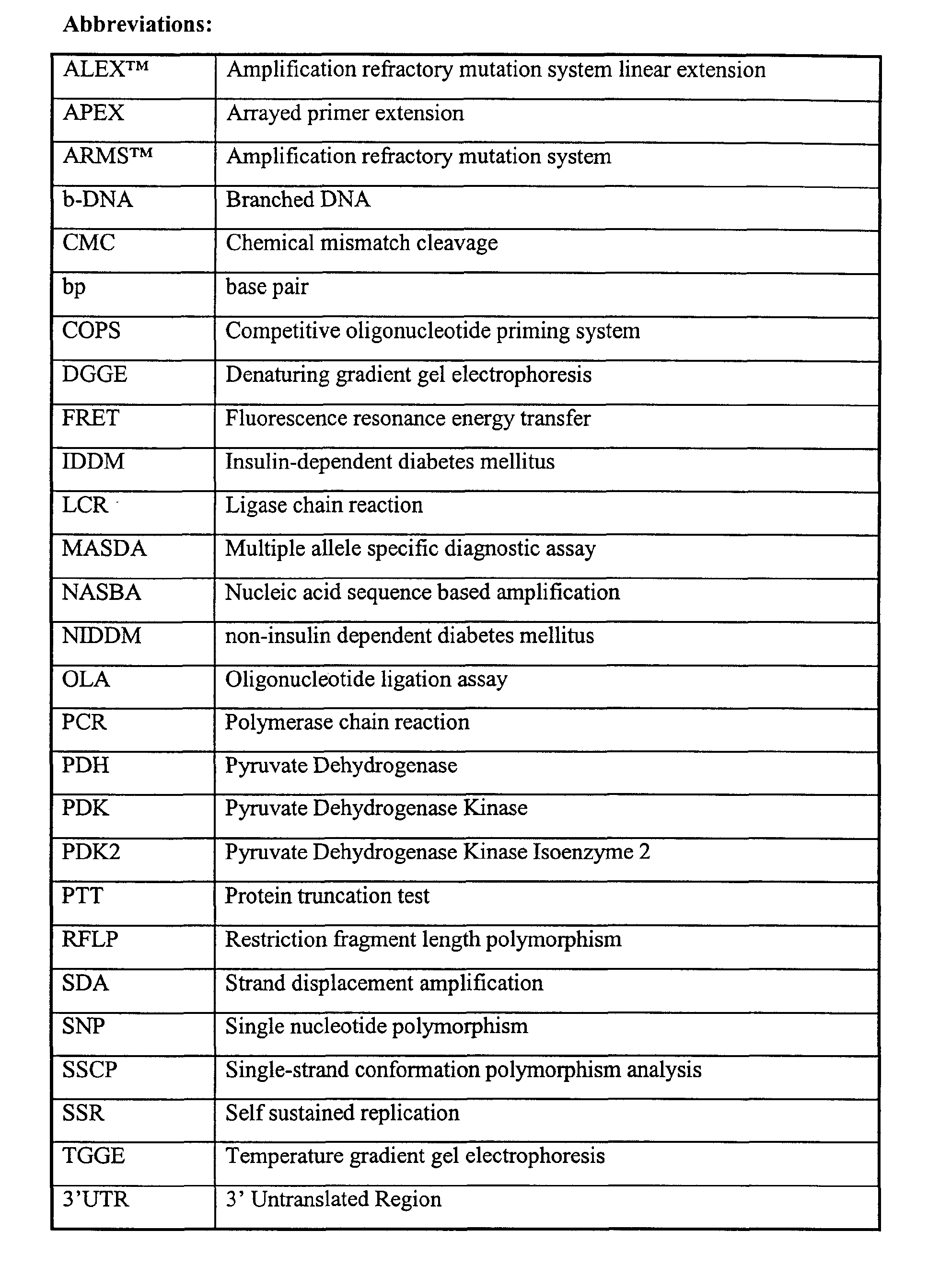

Table 1 - Mutation Detection Techniques
**General:** DNA sequencing, Sequencing by hybridisation
**Scanning:** PTT^{*}, SSCP, DGGE, TGGE, Cleavase, Heteroduplex analysis, CMC, Enzymatic mismatch cleavage ^{*} Note: not useful for detection of promoter polymorphisms.
**Hybridisation Based:**
Solid phase hybridisation: Dot blots, MASDA, Reverse dot blots, Oligonucleotide arrays (DNA Chips).
Solution phase hybridisation: Taqman™ - US-5210015 & US-5487972 (Hoffmann-La Roche), Molecular Beacons - Tyagi *et al* (1996), Nature Biotechnology, **14**, 303; WO 95/13399 (Public Health Inst., New York).
**Extension Based:** ARMS™, ALEX™ - European Patent No. EP 332435 B1 (Zeneca Limited), COPS - Gibbs *et al* (1989), Nucleic Acids Research, **17**, 2347.
**Incorporation Based:** Mini-sequencing, APEX
**Restriction Enzyme Based:** RFLP, Restriction site generating PCR
**Ligation Based:** OLA
**Other:** Invader assay

Table 2 - Signal Generation or Detection Systems
**Fluorescence:** FRET, Fluorescence quenching, Fluorescence polarisation - United Kingdom Patent No. 2228998 (Zeneca Limited)
**Other:** Chemiluminescence, Electrochemiluminescence, Raman, Radioactivity, Colorimetric, Hybridisation protection assay, Mass spectrometry.

Table 3 - Further Amplification Methods
SSR, NASBA, LCR, SDA, b-DNA

Preferred mutation detection techniques include ARMS™, ALEX™, COPS, Taqman, Molecular Beacons, RFLP, restriction site based PCR and FRET techniques, polyacrylamide gel electrophoresis and capillary electrophoresis.

Particularly preferred methods include ARMS™ and RFLP based methods, polyacrylamide gel electrophoresis and capillary electrophoresis. ARMS™, polyacrylamide gel electrophoresis and capillary electrophoresis are especially preferred methods.

In a further aspect, the diagnostic methods of the invention are used to assess the efficacy of therapeutic compounds in the treatment of PDH-mediated diseases such as diabetes, asthma, obesity, sepsis, and peripheral vascular disease.

Assays, for example reporter-based assays, may be devised to detect whether one or more of the above polymorphisms affect transcription levels and/or message stability.

Individuals who carry particular allelic variants of the PDH E1α gene may therefore exhibit differences in their ability to regulate protein biosynthesis under different physiological conditions and may display altered abilities to react to different diseases. In addition, differences in protein regulation arising as a result of allelic variation may have a direct effect on the response of an individual to drug therapy. The diagnostic methods of the invention may be useful both to predict the clinical response to such agents and to determine therapeutic dose.

In a further aspect, the diagnostic methods of the invention, are used to assess the predisposition of an individual to diseases mediated by PDH. This may be particularly relevant in the development of diabetes, asthma, obesity, sepsis, and peripheral vascular disease and other diseases which are mediated by PDH. The present invention may be used to recognise individuals who are particularly at risk from developing these conditions.

Low frequency polymorphisms may be particularly useful for haplotyping as described below. A haplotype is a set of alleles found at linked polymorphic sites (such as within a gene) on a single (paternal or maternal) chromosome. If recombination within the gene is random, there may be as many as 2ⁿ haplotypes, where 2 is the number of alleles at each polymorphic position and n is the number of polymorphic positions. One approach to identifying mutations or polymorphisms which are correlated with clinical response is to carry out an association study using all the haplotypes that can be identified in the population of interest. The frequency of each haplotype is limited by the frequency of its rarest allele, so that polymorphisms with low frequency alleles are particularly useful as markers of low frequency haplotypes. As particular mutations or polymorphisms associated with certain clinical features, such as adverse or abnormal events, are likely to be of low frequency within the population, low frequency polymorphisms may be particularly useful in identifying these mutations (for examples see: De Stefano V et al., *Ann Hum Genet* (1998) 62:481-90; and Keightley AM et al., *Blood* (1999) **93**:4277-83).

In a further aspect, the diagnostic methods of the invention are used in the development of new drug therapies which selectively target one or more allelic variants of the PDH E1α gene. Identification of a link between a particular allelic variant and predisposition to disease development or response to drug therapy may have a significant impact on the design of new drugs. Drugs may be designed to regulate the biological activity of variants implicated in the disease process whilst minimising effects on other variants.

In a further diagnostic aspect of the invention the presence or absence of variant nucleotides is detected by reference to the loss or gain of, optionally engineered, sites recognised by restriction enzymes.

According to another aspect of the present invention there is provided a nucleic acid comprising any one of the following polymorphisms: the nucleic acid of SEQ ID NO.1 with (GGCCAA)n at position 26 as defined by the positions in SEQ ID NO.1, where preferably n = 0 - 100, more preferably n = 0 - 20, more preferably n = 1 - 10, more preferably n = 2, 3 or 4;
the nucleic acid of SEQ ID NO.1 with C or A at position 161 as defined by the positions in SEQ ID NO.1;
or a complementary strand thereof or an antisense sequence thereto or a fragment thereof of at least 20 bases comprising at least one polymorphism.

A nucleic acid of the invention comprises the nucleic acid of SEQ ID NO.1 or a sequence at least 85% homologous thereto; or a complementary strand thereof or an antisense sequence thereto or a fragment thereof of at least 20 bases comprising at least one positions 26 or 161.

Fragments are at least 17 bases, more preferably at least 20 bases, more preferably at least 30 bases. Homology is measured according to the Smith-Waterman algorithm.

The scope of the invention does not extend to any nucleic acid as it is found in nature. A nucleic acid of the invention is preferably in isolated form, for example through being at least partially purified from any substance with which it occurs naturally (if any).

Novel sequence disclosed herein, may be used in another embodiment of the invention to regulate expression of the gene in cells by the use of antisense constructs. To enable methods of down-regulating expression of the gene of the present invention in mammalian cells, an example antisense expression construct can be readily constructed for instance using the pREP10 vector (Invitrogen Corporation). Transcripts are expected to inhibit translation of the gene in cells transfected with this type of construct. Antisense transcripts are effective for inhibiting translation of the native gene transcript, and capable of inducing the effects (e.g., regulation of tissue physiology) herein described. Oligonucleotides which are complementary to and hybridisable with any portion of novel gene mRNA disclosed herein are contemplated for therapeutic use. U.S. Patent No. 5,639,595, "Identification of Novel Drugs and Reagents", issued Jun. 17, 1997, wherein methods of identifying oligonucleotide sequences that display in vivo activity are thoroughly described, is herein incorporated by reference. Expression vectors containing random oligonucleotide sequences derived from previously known polynucleotides are transformed into cells. The cells are then assayed for a phenotype resulting from the desired activity of the oligonucleotide. Once cells with the desired phenotype have been identified, the sequence of the oligonucleotide having the desired activity can be identified. Identification may be accomplished by recovering the vector or by polymerase chain reaction (PCR) amplification and sequencing the region containing the inserted nucleic acid material. Antisense molecules can be synthesised for antisense therapy. These antisense molecules may be DNA, stable derivatives of DNA such as phosphorothioates or methylphosphonates, RNA, stable derivatives of RNA such as 2'-O-alkylRNA, or other oligonucleotide mimetics. U.S. Patent No. 5,652,355, "Hybrid Oligonucleotide Phosphorothioates", issued July 29, 1997, and U.S. Patent No. 5,652,356, "Inverted Chimeric and Hybrid Oligonucleotides", issued July 29, 1997, which describe the synthesis and effect of physiologically-stable antisense molecules, are incorporated by reference. Antisense molecules may be introduced into cells by microinjection, liposome encapsulation or by expression from vectors harboring the antisense sequence.

The invention further provides nucleotide primers which can detect the polymorphisms of the invention.

According to another aspect of the present invention there is provided an allele specific primer capable of detecting a PDH E1α gene polymorphism at one or more of position 1388 in the PDH E1α gene as defined by the position in SEQ ID NO: 2 and/or positions 26 and 161 of intron 7 of the PDH E1α gene as defined by the positions in SEQ ID NO.1.

An allele specific primer is used, generally together with a constant primer, in an amplification reaction such as a PCR reaction, which provides the discrimination between alleles through selective amplification of one allele at a particular sequence position e.g. as used for ARMS™ assays. The allele specific primer is preferably 17- 50 nucleotides, more preferably about 17-35 nucleotides, more preferably about 17-30 nucleotides.

An allele specific primer preferably corresponds exactly with the allele to be detected but derivatives thereof are also contemplated wherein about 6-8 of the nucleotides at the 3' terminus correspond with the allele to be detected and wherein up to 10, such as up to 8, 6, 4, 2, or 1 of the remaining nucleotides may be varied without significantly affecting the properties of the primer.

Primers may be manufactured using any convenient method of synthesis. Examples of such methods may be found in standard textbooks, for example "Protocols for Oligonucleotides and Analogues; Synthesis and Properties," Methods in Molecular Biology Series; Volume 20; Ed. Sudhir Agrawal, Humana ISBN: 0-89603-247-7; 1993; 1^{st} Edition. If required the primer(s) may be labelled to facilitate detection.

According to another aspect of the present invention there is provided an allele-specific oligonucleotide probe capable of detecting a PDH E1α gene polymorphism at one or more of position 1388 in the PDH Elα gene as defined by the position in SEQ ID NO: 2 and/or positions 26 and 161 of intron 7 of the PDH E1α gene as defined by the positions in SEQ ID NO.1.

The allele-specific oligonucleotide probe is preferably 17- 50 nucleotides, more preferably about 17-35 nucleotides, more preferably about 17-30 nucleotides.

The design of such probes will be apparent to the molecular biologist of ordinary skill. Such probes are of any convenient length such as up to 50 bases, up to 40 bases, more conveniently up to 30 bases in length, such as for example 8-25 or 8-15 bases in length. In general such probes will comprise base sequences entirely complementary to the corresponding wild type or variant locus in the gene. However, if required one or more mismatches may be introduced, provided that the discriminatory power of the oligonucleotide probe is not unduly affected. The probes of the invention may carry one or more labels to facilitate detection.

According to another aspect of the present invention there is provided a diagnostic kit comprising an allele specific oligonucleotide probe of the invention and/or an allele-specific primer of the invention.

The diagnostic kits may comprise appropriate packaging and instructions for use in the methods of the invention. Such kits may further comprise appropriate buffer(s), nucleotides, and polymerase(s) such as thermostable polymerases, for example taq polymerase.

In another aspect of the invention, any of the polymorphisms or haplotype of this invention may be used as a genetic marker in a linkage study.

According to another aspect of the present invention there is provided a method of treating a human in need of treatment with a PDH drug in which the method comprises:
i) diagnosis of a polymorphism in the PDH E1α gene in the human, which diagnosis comprises determining the sequence of the nucleic acid at one or more of position 1388 in the PDH Elα gene as defined by the position in SEQ ID NO: 2 and/or positions 26 and 161 of intron 7 of the PDH Elα gene as defined by the positions in SEQ ID NO.1, and determining the status of the human by reference to polymorphism in the PDH E1α gene; and
ii) administering an effective amount of a PDH drug.

Preferably determination of the status of the human is clinically useful. Examples of clinical usefulness include deciding which drug or drugs to administer and/or establishing the effective amount of the drug or drugs.

Drugs which increase the activity of PDH are of value in a number of disease conditions, including disease states associated with disorders of glucose utilisation such as diabetes and obesity, and associated with excessive production of lactate such as encountered in sepsis and other causes of lactic acidaemia. Additionally drugs which increase the activity of PDH may be expected to have utility in diseases where supply of energy-rich substrates to tissues is limiting such as peripheral vascular disease, coronary failure and certain cardiac myopathies, muscle ataxia and weakness.

According to another aspect of the present invention there is provided use of a PDH drug in the preparation of a medicament for treating a PDH-mediated disease in a human diagnosed as having a polymorphism at one or more of position 1388 in the PDH E1α gene as defined by the position in SEQ ID NO: 2 and/or positions 26 and 161 of intron 7 of the PDH E1α gene as defined by the positions in SEQ ID NO.1.

According to another aspect of the present invention there is provided a pharmaceutical pack comprising a PDH drug and instructions for administration of the drug to humans diagnostically tested for a polymorphism at one or more of position 1388 in the PDH E1α gene as defined by the position in SEQ ID NO: 2 and/or positions 26 and 161 of intron 7 of the PDH Elα gene as defined by the positions in SEQ ID NO.1.

SEQ ID NO.1 identifies for the first time, the sequence of intron 7 of the human PDH Elα gene.

Therefore in another aspect of the present invention we provide the genetic sequence of PDH E1α intron 7 as set out in SEQ ID NO.1.

In another aspect of the present invention we provide fragments of PDH Elα intron 7. By fragments we mean contiguous regions of the gene including complementary DNA and RNA sequences, starting with short sequences useful as probes or primers of say about 8-50 bases, such as 10-30 bases or 15-35 bases, to longer sequences of say about 50, 100 or 150 bases up to and including the full length sequence depicted in SEQ ID NO.1. Any convenient fragment of the PDH E1α intron 7 gene may be a useful gene fragment for further research, therapeutic or diagnostic purposes. Further convenient fragments include those whose termini are defined by restriction sites within the gene sequence of one or more kinds.

In another aspect of the present invention we provide homologues of the human PDH Elα intron 7 gene sequence. By homologue we mean a corresponding gene sequence in the same or another species which displays greater than 85% sequence homology, conveniently greater than 90%, for example 95%, compared to the human PDH Elα intron 7 gene sequence set out in SEQ ID NO.1. The full sequence of the individual homologues may be determined using conventional techniques such as hybridisation, PCR and sequencing techniques, starting with any convenient part of the sequence set out in SEQ ID NO.1.

In another aspect of the present invention we provide polynucleotide sequences capable of specifically hybridising to the PDH Elα intron 7 gene sequence. By specifically hybridising we mean that the polynucleotide hybridises under stringent conditions to the sequence set out in SEQ ID NO.1 or to its complementary sequence, to the exclusion of other genetic loci. It is contemplated that a peptide nucleic acid may be an acceptable equivalent to a polynucleotide.

Knowledge of the gene sequence of PDH Elα intron 7 provides the ability to regulate its activity *in vivo,* for example by the use of antisense DNA or RNA.

Therefore, according to another aspect of the present invention we provide an antisense DNA or an antisense RNA which is complementary to the polynucleotide sequence of PDH E1α intron 7, shown in SEQ ID NO.1. By complementary we mean that the two molecules can base pair to form a double stranded molecule. The antisense DNA or RNA for co-operation with the gene in SEQ ID NO.1 may be produced by conventional means, by standard molecular biology or by chemical synthesis as described above. If desired the antisense DNA or antisense RNA may be chemically modified so as to prevent degradation *in vivo* or to facilitate passage through a cell membrane and the invention extends to such constructs.

According to another aspect of the present invention there is provided a computer readable medium comprising at least one novel polynucleotide sequence of the invention stored on the medium. The computer readable medium may be used, for example, in homology searching, mapping, haplotyping, genotyping or pharmacogenetic analysis or any other bioinformatic analysis. The reader is referred to Bioinformatics, A practical guide to the analysis of genes and proteins, Edited by A D Baxevanis & B F F Ouellette, John Wiley & Sons, 1998. Any computer readable medium may be used, for example, compact disk, tape, floppy disk, hard drive or computer chips.

The polynucleotide sequences of the invention, or parts thereof, particularly those relating to and identifying the polymorphisms identified herein represent a valuable information source, for example, to characterise individuals in terms of haplotype and other sub-groupings, such as investigation of susceptibility to treatment with particular drugs. These approaches are most easily facilitated by storing the sequence information in a computer readable medium and then using the information in standard bioinformatics programs or to search sequence databases using state of the art searching tools such as "GCC". Thus, the polynucleotide sequences of the invention are particularly useful as components in databases useful for sequence identity and other search analyses. As used herein, storage of the sequence information in a computer readable medium and use in sequence databases in relation to 'polynucleotide or polynucleotide sequence of the invention' covers any detectable chemical or physical characteristic of a polynucleotide of the invention that may be reduced to, converted into or stored in a tangible medium, such as a computer disk, preferably in a computer readable form. For example, chromatographic scan data or peak data, photographic scan or peak data, mass spectrographic data, sequence gel (or other) data.

The invention provides a computer readable medium having stored thereon one or more polynucleotide sequences of the invention. For example, a computer readable medium is provided comprising and having stored thereon a member selected from the group consisting of: a polynucleotide comprising the sequence of a polynucleotide of the invention, a polynucleotide consisting of a polynucleotide of the invention, a polynucleotide which comprises part of a polynucleotide of the invention, which part includes at least one of the polymorphisms of the invention, a set of polynucleotide sequences wherein the set includes at least one polynucleotide sequence of the invention, a data set comprising or consisting of a polynucleotide sequence of the invention or a part thereof comprising at least one of the polymorphisms identified herein.

A computer based method is also provided for performing sequence identification, said method comprising the steps of providing a polynucleotide sequence comprising a polymorphism of the invention in a computer readable medium; arid comparing said polymorphism containing polynucleotide sequence to at least one other polynucleotide or polypeptide sequence to identify identity (homology), i.e. screen for the presence of a polymorphism.

Another aspect of the invention is use of any one of the following in bioinformatic analysis:
i) any polymorphism as defined above;
ii) the haplotype defined above; or
iii) a novel nucleic acid sequence as defined above.
Preferably the use comprises a bioinformatic analysis selected from homology searching, mapping, haplotyping, genotyping or pharmacogenetic analysis.

The invention will now be illustrated but not limited by reference to the following Examples. All temperatures are in degrees Celsius.

In the Examples below, unless otherwise stated, the following methodology and materials have been applied.

AMPLITAQ™ or AMPLITAQ GOLD™ available from Perkin-Elmer Cetus, are used as the source of thermostable DNA polymerase.

General molecular biology procedures can be followed from any of the methods described in "Molecular Cloning - A Laboratory Manual" Second Edition, Sambrook, Fritsch and Maniatis (Cold Spring Harbor Laboratory, 1989).

Electropherograms were obtained in a standard manner: data was collected by ABI377 data collection software and the wave form generated by ABI Prism™ sequencing analysis (2.1.2).

### Example 1

### Identification of Polymorphisms

### 1. Methods

### c-DNA Preparation

RNA was prepared from lymphoblastoid cell lines from Caucasian donors using standard laboratory protocols (Chomczynski and Sacchi, Anal. Biochem. 162, 156-159, 1987) and used to generate first strand cDNA (Gubler and Hoffman, Gene 25, 263-269, 1983).

### DNA Preparation

DNA was prepared from the same lymphoblastoid cell lines following protocol I (Molecular Cloning: A Laboraotory Manual, p392, Sambrook, Fritsch and Maniatis, 2nd Edition, Cold Spring Harbor Press, 1989) with the following modifications. Samples were extracted with phenol, then phenol/chloroform and then chloroform rather than with three phenol extractions. The DNA was dissolved in deionised water.

### Template Preparation

Templates were prepared by PCR using the oligonucleotide primers and annealing temperatures set out below. The extension temperature was 72° and denaturation temperature 94°; each step was 1 minute. Generally 100 pg cDNA or 50 ng genomic DNA was used in each reaction and subjected to 40 cycles of PCR.

| **Fragment** | **Forward Oligo** | **Reverse Oligo** | **Annealing Temp** | **MgCl**_{**2**} |
|---|---|---|---|---|
| 1-526 | 1-20 | 505-526 | 60° | 1mM |
| 310-741 | 310-331 | 720-741 | 64° | 1mM |
| 632-1111 | 632-653 | 1089-1111 | 62° | 1mM |
| 995-1430 | 995-1015 | 1409-1430 | 60° | 1mM |
| 841-936 | 841-862 | 916-936 | 62° | 1mM |

Fragment 841-936 was prepared from genomic DNA and includes intron 7.

For dye-primer sequencing the forward primers were modified to include M13 forward sequence (ABI protocol P/N 402114, Applied Biosystems) at the 5' end of the oligonucleotides.

### Dye Primer Sequencing

Dye-primer sequencing using M13 forward primer was as described in the ABI protocol P/N 402114 for the ABI Prism™ dye primer cycle sequencing core kit with "AmpliTaq FS"™ DNA polymerase, modified in that the annealing temperature was 45° and DMSO was added to the cycle sequencing mix to a final concentration of 5%. The extension reactions for each base were pooled, ethanol/sodium acetate precipitated, washed and resuspended in formamide loading buffer.

4.25% Acrylamide gels were run on an automated sequencer (ABI 377, Applied Biosystems).

### 2. Results

### Novel Polymorphisms

| ***Position*** | ***Reference*** | ***Region*** | ***Allele 1*** | ***Allele 2*** | ***RFLP*** | ***Frequency*** |
|---|---|---|---|---|---|---|
| 161 | SEQ ID NO.1 | intron 7 | C | A | No | 4/56 |
| 1388 | SEQ ID NO.2 | 3' UTR | C | T | No | 3/23 |

Frequency is the allele frequency of allele 2 in control subjects.

| ***Position*** | ***Reference*** | ***Region*** | ***Polymorphism*** | ***Frequency*** |
|---|---|---|---|---|
| 26 | SEQ ID NO.1 | intron 7 | (GGCCAA)n | n=2 4/56 |
| | | | | n=3 49/56 |
| | | | | n=4 3/56 |

A at position 161 of intron 7 was only seen on the 4/56 chromosomes which carried the (GGCCAA)₂ allele.

### SEQ ID NO.1

### Intron 7 of the human PDH E1α gene.

The hexanucleotide repeat starting at position 26 (GGCCAA)n is shown with n=2.

GTAAGGACAC CTGTGGTGGG GCCGGGGCCA AGGCCAAGGG TATGTCCTTG
TGCAGACCCT TGACGATCTT AGAAACATTG GAGAGTTTCA TTCTCATACA
GGAGCAGGTC ATGTGAAAGT AAAATGGTTT GGGGCAGTTG GATTCATGCT
TCGCCCCTCC CCTGTTTATT ACCAG

## Claims

1. A method for the diagnosis of a polymorphism in a PDH E1α gene in a human, which method comprises determining the sequence of the nucleic acid of the human at position 1388 in the PDH Elα gene as defined by the position in SEQ ID NO: 2, and/or at one or more of positions 26 and 161 of intron 7 of the PDH Elα gene as defined in SEQ ID NO.1; and determining the status of the human by reference to polymorphism in the PDH Elα gene.

2. A method according to claim 1 in which the polymorphisms are further defined as:
| **Position** | **Reference** | **Region** | **Polymorphism** |
|---|---|---|---|
| 26 | SEQ ID NO: 1 | intron 7 | (GGCCAA)n |
| 161 | SEQ ID NO:1 | intron 7 | C/A |
| 1388 | SEQ ID NO:2 | 3'UTR | C/T |

3. A method according to claim 2 which comprises diagnosis of the following haplotype:
| **Position** | **Reference** | **Region** | **Polymorphism** |
|---|---|---|---|
| 26 | SEQ ID NO: 1 | intron 7 | (GGCCAA)₂ |
| 161 | SEQ ID NO:1 | intron 7 | A |

4. A nucleic acid comprising the nucleic acid of SEQ ID NO.1 or a sequence at least 85% homologous thereto; or a complementary strand thereof or an antisense sequence thereto or a fragment thereof of at least 20 bases comprising at least one positions 26 or 161.

5. An allele specific primer capable of detecting a PDH Elα gene polymorphism at one or more of position 1388 in the PDH Elα gene as defined by the position in SEQ ID NO: 2 and/or positions 26 and 161 of intron 7 of the PDH Elα gene as defined by the positions in SEQ ID NO.1.

6. An allele-specific oligonucleotide probe capable of detecting a PDH Elα gene polymorphism at one or more of position 1388 in the PDH Elα gene as defined by the position in SEQ ID NO: 2 and/or positions 26 and 161 of intron 7 of the PDH Elα gene as defined by the positions in SEQ ID NO.1.

7. Use of any polymorphism as defined in claim 2 as a genetic marker in a linkage study.

8. Use of a PDH drug in the preparation of a medicament for treating a PDH-mediated disease in a human diagnosed as having a polymorphism at one or more of position 1388 in the PDH Elα gene as defined by the position in SEQ ID NO: 2 and/or positions 26 and 161 of intron 7 of the PDH Elα gene as defined by the positions in SEQ ID NO.1.

9. Use of any one of the following in bioinformatic analysis:
i) any polymorphism as defined in claim 1 or 2;
ii) the haplotype defined in claim 3; or
iii) a nucleic acid sequence as defined in claim 4.

10. A use according to claim 9 comprising a bioinformatic analysis selected from homology searching, mapping, haplotyping, genotyping or pharmacogenetic analysis.
